# EUROPÄISCHE PATENTSCHRIFT

(11) **EP 2 113 263 B1**
(45) Veröffentlichungstag und Bekanntmachung des Hinweises auf die Patenterteilung: **17.06.2015**
(21) Anmeldenummer: 09005196.2
(22) Anmeldetag: 09.04.2009
(51) Int. Cl.: A61L 27/36

(54) **Implantat, insbesondere zur Wiederherstellung und/oder Regenerierung von menschlichem und/oder tierischem Gewebe**
Implant, in particular for restoring and/or regenerating human and/or animal tissue
Implant, notamment pour la reproduction et/ou la régénération de tissus humains et/ou animaux

(30) Priorität: 30.04.2008 DE 102008022319
(43) Veröffentlichungstag der Anmeldung: 04.11.2009
(73) Patentinhaber: Aesculap AG, 78532 Tuttlingen/Donau (DE)
(72) Erfinder: Scholl, Edmund, 78532 Tuttlingen (DE); Kalb, Manfred, 34212 Melsungen-Obermelsungen (DE)
(74) Vertreter: Patentanwälte Ruff, Wilhelm, Beier, Dauster & Partner

(56) Entgegenhaltungen:
- WO-A2-99/62425
- WO-A2-2005/023321
- DE-A1-102005 054 940
- US-A1- 2005 249 771

## Beschreibung

Die Erfindung betrifft ein Implantat, welches sich vorzugsweise zur Wiederherstellung und/oder Regenerierung von menschlichem und/oder tierischem Gewebe eignet, und ein Herstellungsverfahren für das Implantat.

Zur Behandlung von Gewebedefekten werden heutzutage in verstärktem Maße Implantate auf Kollagenbasis eingesetzt. Beispiele für derartige Kollagenimplantate sind die unter den Bezeichnungen Lyostypt^{®} und Lyoplant^{®} vertriebenen Erzeugnisse der Anmelderin. Eine Kollagenfolie zur Reparatur und Regenerierung von Gewebe der Dura Mater ist beispielsweise aus der US 2006/0167561 A1 bekannt. In der US 5,997,895 wird eine Kollagenmatrix zur Behandlung von beschädigtem Hirnhautgewebe offenbart.

Ein weiteres Kollagenimplantat geht aus der US 6,977,231 B1 hervor. Bei dem dort beschriebenen Implantat handelt es sich um eine Kollagenmembran, welche zumindest eine non-woven Schicht aus Kollagenfasern und zumindest eine schwammartige Schicht aus Kollagen aufweist, wobei die Membran an ihrer Oberfläche eine Gelatine- oder Hyaluronsäurebeschichtung aufweist. Die Gelatine- bzw. Hyaluronsäurebeschichtung soll dabei Gewebeverklebungen vorbeugen. Ein weiteres Beispiel für ein Kollagenimplantat ist das von der Anmelderin unter der Bezeichnung Novocart 3D kommerziell vertriebene Erzeugnis.

In der US 2005/0249771 A1 wird ein bioprothetisches Verbundmaterial zur Unterstützung der Gewebsregeneration beschrieben. Ausgehend von einem synthetischen Grundgerüst erfolgt eine ein- oder beidseitige Beschichtung mit kollagenhaltigem Gewebsmaterial wie beispielsweise Dünndarmschleimhaut.

Ein weiteres Komposit-Implantat auf Basis von zwei extrazellulären Matrixmaterialien ist in der WO 2005/023321 A2 offenbart, wobei auf einem ersten zellfreien extrazellulären Matrixmaterial durch zelluläre Abscheidung eine zweite extrazelluläre Matrixschicht generiert wird.

Die WO 99/62425 A2 beschreibt eine ein- oder mehrschichtige tubuläre Prothese aus intestinalem Kollagenmaterial. Das Hohlkörperimplantat kann zusätzlich auf seiner Innen- bzw. Außenseite mit einer Schicht fibrilären Kollagens versehen sein.

Nachteilig bei den aus dem Stand der Technik bekannten Implantaten ist, dass sie häufig aus unterschiedlichen Ausgangsmaterialien hergestellt werden. Dies führt zum einen zu einem aufwendigeren Herstellungsprozess. Zum anderen können sich dadurch auch behördliche Genehmigungsverfahren zum Teil erheblich verzögern. Insgesamt kann sich dadurch der Zeit- und Kostenaufwand für die Herstellung und die medizinische Validierung von Implantaten vervielfachen.

Die vorliegende Erfindung stellt sich daher die Aufgabe, ein im Vergleich zu den aus dem Stand der Technik bekannten Implantaten alternatives Implantat bereitzustellen, welches einerseits über wundversorgende und -versiegelnde Eigenschaften andererseits über eine gewisse mechanische Robustheit verfügen sollte. Darüber hinaus sollte das Implantat möglichst einfach in der Herstellung sowie möglichst unproblematisch bezüglich seiner medizinischen Zulassung sein.

Diese Aufgabe wird erfindungsgemäß gelöst durch ein implantat, insbesondere zur Wiederherstellung und/oder Regenerierung von menschlichem und/oder tierischem Gewebe, mit einem schichtförmigen Aufbau, umfassend zumindest eine erste und eine zweite Pericardschicht. Grundsätzlich kann das Implantat einen mehrschichtigen Aufbau aufweisen. Insbesondere kann das Implantat eine alternierende Abfolge aus der ersten und der zweiten Pericardschicht aufweisen. Weiterhin kann das Implantat eine Sandwichstruktur aufweisen, bei der beispielsweise beide Seiten der ersten Pericardschicht mit der zweiten Pericardschicht belegt sind. Bevorzugt ist ein zweischichtiger Aufbau aus der ersten und der zweiten Pericardschicht. Das Implantat kann weiterhin überstehende Ränder aufweisen. Erfindungsgemäß kann die erste oder die zweite Pericardschicht im Implantat überstehende Ränder aufweisen.

In einer bevorzugten Ausführungsform berühren sich die erste und die zweite Pericardschicht entlang einer gemeinsamen Grenzfläche. In der Regel ist die erste Pericardschicht vollflächig auf der zweiten Pericardschicht bzw. die zweite Pericardschicht vollflächig auf der ersten Pericardschicht ausgebildet.

Erfindungsgemäß sind die erste und die zweite Pericardschicht unterschiedlich ausgebildet. So kann die erste Pericardschicht eine im Wesentlichen feste und insbesondere lederartige Beschaffenheit aufweisen. In dieser Ausführungsform besitzt die erste Pericardschicht lasttragende Eigenschaften. Vorzugsweise stellt die erste Pericardschicht eine Art Trägermaterial für die zweite Pericardschicht dar. Dadurch kann das Implantat über die erste Pericardschicht an der zu versorgenden Körperregion befestigt werden. Zur Befestigung kann das Implantat an der jeweiligen Körperstelle vernäht oder verklebt werden. Erfindungsgemäß ist die erste Pericardschicht eine Pericardfolie.

In einer weiteren Ausführungsform weist die erste Pericardschicht einen Anteil an Pericardium fibrosum und einen Anteil an Pericardium serosum auf. Bevorzugt sind diese Pericardanteile schichtförmig in der ersten Pericardschicht angeordnet. So kann die erste Pericardschicht insbesondere eine Unterschicht aus Pericardium fibrosum und eine Unterschicht aus Pericardium serosum aufweisen. In einer bevorzugten Ausführungsform ist die Unterschicht aus Pericardium fibrosum auf einer Seite, d.h. einseitig, von der zweiten Pericardschicht vorzugsweise vollflächig bedeckt bzw. mit der zweiten Pericardschicht vorzugsweise vollflächig belegt. Weiterhin ist die andere Seite der Unterschicht aus Pericardium fibrosum bevorzugt mit der Unterschicht aus Fibrosum serosum belegt.

Die zweite Pericardschicht ist in einer weiteren geeigneten Ausführungsform aus homogenisierten, insbesondere aus in flüssiger Dispersion homogenisierten, Pericardstücken gebildet. Die zweite Pericardschicht ist schwammartig ausgebildet. Durch die Ausbildung als Schwamm besitzt die zweite Pericardschicht mit besonderem Vorteil saugfähige Eigenschaften gegenüber Körperflüssigkeiten, beispielsweise Blut, Lymphe, Exsudat oder Eiterflüssigkeiten. Durch das Eindringen von Körperflüssigkeiten in die zweite Pericardschicht wird zum einen das Wundareal getrocknet, was insgesamt zu einer beschleunigten Heilung führt. Zum anderen erhält die zweite Pericardschicht durch ihr Aufsaugen von Körperflüssigkeiten mit Vorteil eine hydrogelartige Konsistenz, wodurch das Implantat insgesamt gewebeverklebende Eigenschaften erhält. Gemäß einer besonders bevorzugten Ausführungsform ist das Implantat selbstklebend, so dass zusätzliche Fixierungsschritte, wie beispielsweise Annähen oder Ankleben mit einem hierfür vorgesehenen Kleber, entfallen.

In einer weiteren Ausführungsform liegen die erste und/oder die zweite Pericardschicht, vorzugsweise die erste und die zweite Pericardschicht, in lyophilisierter Form vor. Erfindungsgemäß kann es insbesondere vorgesehen sein, dass die zweite Pericardschicht auf die erste Pericardschicht auflyophilisiert ist.

In einer besonders bevorzugten Ausführungsform ist die erste Pericardschicht eine lyophilisierte Pericardfolie und die zweite Pericardschicht eine schwammartige, lyophilisierte Pericardschicht. Überraschenderweise hat sich herausgestellt, dass ein Implantat in Form eines zweischichtigen Verbundes aus Pericardfolie und -schwamm besonders stabil ist und insbesondere in Anwesenheit von Wasser oder physiologischen Flüssigkeiten nicht zerstört wird.

Das Pericard der ersten und/oder zweiten Pericardschicht kann grundsätzlich humanen Ursprungs sein. Vorzugsweise ist das Pericard jedoch xenogenen, insbesondere bovinen, porcinen und/oder equinen, Ursprungs. Bevorzugt ist das Pericard der ersten und zweiten Pericardschicht gleichen biologischen Ursprungs. Besonders bevorzugt handelt es sich bei dem Pericard der ersten und/oder zweiten Schicht um Rinderpericard. Das Pericard kann weiterhin grundsätzlich Kollagen vom Typ I und/oder Kollagen vom Typ III aufweisen. Bevorzugt ist Kollagen vom Typ I.

Das Pericard stammt gewöhnlich aus einem aufbereiteten Pericardgewebe. Das Pericard kann dabei nasschemisch aufbereitet, oxidativ gebleicht, ausgewaschen, entfettet, getrocknet und/oder sterilisiert vorliegen.

Die erste Pericardschicht weist in einer weiteren Ausführungsform eine Schichtdicke zwischen 0.1 und 2.0 mm, insbesondere 0.5 und 1.5 mm, auf. Die erste Pericardschicht kann ein Flächengewicht zwischen 50 und 250 g/m², vorzugsweise 100 und 180 g/m², aufweisen. Weiterhin ist es bevorzugt, dass die erste Pericardschicht einen Anteil zwischen 20 und 80 Gew.-%, insbesondere 40 und 60 Gew.-%, bezogen auf das Gesamtgewicht des Implantats, aufweist.

Die zweite Pericardschicht weist vorzugsweise eine Schichtdicke zwischen 0.5 und 4 mm, insbesondere 2 und 3 mm, auf. Die zweite Pericardschicht kann insbesondere ein Flächengewicht zwischen 25 und 200 g/m², vorzugsweise 80 und 120 g/m², aufweisen. Bei einer weiteren Ausführungsform weist die zweite Pericardschicht einen Anteil zwischen 20 und 80 Gew.-%, bezogen auf das Gesamtgewicht des Implantats, auf.

In einer weiteren Ausführungsform weist das Implantat eine Dichte zwischen 12 und 180 mg/cm³, bevorzugt 40 und 80 mg/cm³, auf. Die zweite Pericardschicht kann weiterhin einen Porendurchmesser zwischen 20 und 150 µm, insbesondere 80 und 120 µm, aufweisen. Besonders bevorzugt weist die zweite Pericardschicht ein Aufnahmevermögen für Körperflüssigkeiten, insbesondere Blut, Lymphe, Exsudat und/oder Eiterflüssigkeiten, auf, das dem 5- bis 25-fachen, insbesondere 10- bis 20-fachen, ihres trockenen Eigengewichtes entspricht.

Zur Erhöhung der Stabilität des Implantats kann insbesondere die zweite Pericardschicht vernetzt vorliegen. Grundsätzlich kann es sich bei der Vernetzung um eine physikalische Vernetzung handeln. Bevorzugt ist die zweite Pericardschicht jedoch chemisch vernetzt. Als Vernetzungsmittel kommen allgemein bioverträgliche Verbindungen in Betracht. Zweckmäßigerweise weist das Implantat dabei einen Anteil an Vernetzungsmitteln auf, der unter medizinischen Gesichtspunkten zu keiner nennenswerten Gewebeschädigung führt. Bei den Vernetzungsmitteln handelt es sich gewöhnlich um multifunktionelle, insbesondere bifunktionelle, Verbindungen. Als Vernetzungsmittel können zum Beispiel Diamine, Carbodiimide, Diisocyanate, Dicarbonsäuren, Di- und/oder Polyaldehyde verwendet werden. Bei den Polyaldehyden handelt es sich bevorzugt um Aldehydgruppen tragende (polyaldehydische) Polysaccharide

Die polyaldehydischen Polysaccharide weisen vorzugsweise einen Oxidationsgrad von < 50%, insbesondere < 35%, vorzugsweise < 25%, auf. Unter dem Oxidationsgrad soll hierbei der Anteil an Aldehydgruppen und gegebenenfalls Carboxylgruppen tragenden Monosaccharideinheiten im polyaldehydischen Polysaccharid verstanden werden. Die Verwendung von Aldehydgruppen tragenden Polysacchariden mit niedrigeren Oxidationsgraden ist wegen ihrer schnelleren Resorbierbarkeit besonders bevorzugt. Erfindungsgemäß können die polyaldehydischen Polysaccharide frei von Carboxylgruppen sein. Bei dem Carbodiimid kann es sich beispielsweise um EDC (1-Ethyl-3-(3-Dimethylaminopropyl)carbodiimid-hydrochlorid) handeln. Ein weiteres geeignetes Vernetzungsmittel ist n-Hydroxysuccinimid (NHS).

Bei einer weiteren Ausführungsform weist das Implantat, insbesondere die zweite Pericardschicht, Wirkstoffe auf. In dieser Ausführungsform dient die zweite Pericardschicht bevorzugt als Wirkstoffdepot. Nach der Implanta-tion können die Wirkstoffe aus dem Implantat herausdiffundieren und in die unmittelbare Umgebung der Implantationsstelle gelangen und auf diese Weise insgesamt zu einem beschleunigten Heilungsprozess beitragen. Bei den Wirkstoffen selbst kann es sich allgemein um Arzneistoffe und/oder biologische Wirkstoffe, beispielsweise Hormone und/oder Wachstumsfaktoren, handeln. Bevorzugt sind die Wirkstoffe aus der Gruppe antimikrobielle Verbindungen, entzündungshemmende Verbindungen, blutstillende Verbindungen und Wachstumsfaktoren ausgewählt. Weiterhin kann das Implantat, insbesondere die zweite Pericardschicht, Körperzellen, vorzugsweise autologe Körperzellen, enthalten.

In einer weiteren Ausführungsform ist zumindest eine Pericardschicht gefärbt. Beispielsweise kann die zweite Pericardschicht mit zumindest einem Farbstoff gefärbt sein. Bei dem zumindest einen Farbstoff kann es sich beispielsweise um D- & C-Farbstoffe (drug- und cosmetic-Farbstoffe), Riboflavin, Retinol und/oder Methylenblau handeln. Die Färbung der Pericardschicht bzw. Pericardschichten dient vor allem einer leichteren Unterscheidbarkeit der ersten und der zweiten Pericardschicht für den Anwender, insbesondere Chirurgen.

Das Implantat kann abhängig vom jeweiligen Verwendungszweck in unterschiedlichen Formen und Dimensionen vorliegen. Gewöhnlich handelt es sich bei dem erfindungsgemäßen Implantat um ein flächiges Implantat. Das Implantat liegt weiterhin zweckmäßigerweise in sterilisierter Form vor. Als Sterilisationsmethoden kommen alle dem Fachmann bekannten Verfahren, insbesondere Gammasterilisierung, Ethylenoxidbegasung oder Plasmasterilisierung, in Frage. Das Implantat kann außerdem in konfektionierter Form vorliegen.

Die vorliegende Erfindung betrifft auch ein Verfahren zur Herstellung des Implantats, wobei zumindest eine erste Pericardschicht mit einer zweiten Pericardschicht beschichtet wird.

Das für die Herstellung des Implantats verwendete Pericard wird in der Regel durch Aufarbeitung von rohem Pericard-Gewebe, vorzugsweise Rinder-Pericard-Gewebe, gewonnen. Die Aufarbeitung des rohen Pericard-Gewebes erfolgt bevorzugt durch eine nasschemische Aufbereitung, oxidative Bleichung, Auswaschung, Entfettung, Trocknung und/oder Sterilisierung. Bezüglich weiterer Merkmale und Einzelheiten für eine mögliche Aufarbeitung von rohem Pericard-Gewebe wird auf die DE 38 35 237 C1 verwiesen, deren Offenbarungsgehalt insoweit durch ausdrückliche Bezugnahme zum Inhalt der vorliegenden Beschreibung gemacht wird.

In einer bevorzugten Ausführungsform wird für die Beschichtung der ersten Pericardschicht
a) die erste Pericardschicht mit einer flüssigen Dispersion, umfassend Pericardstücke und ein Dispersionsmittel, in Kontakt gebracht und
b) anschließend das Dispersionsmittel unter Ausbildung der zweiten Pericardschicht entfernt.

Zur Herstellung der flüssigen Dispersion wird in der Regel eine wässrige Flüssigkeit, insbesondere Wasser oder eine Pufferlösung, verwendet. Bevorzugt lässt man die Pericardstücke in dem Dispersionsmittel vorquellen. Die Pericardstücke werden in einer geeigneten Ausführungsform in der Dispersion homogenisiert, ehe die erste Pericardschicht mit der Dispersion in Kontakt gebracht wird. Die Homogenisierung der Pericardstücke erfolgt gewöhnlich durch mechanisches Zerkleinern, beispielsweise mit einem Messer eines Dispergiergerätes.

Für das Inkontaktbringen mit der Pericard-Dispersion wird die erste Pericardschicht bei einer weiteren Ausführungsform in eine formgebende Umgebung, beispielsweise in eine Lyophilisationsplatte, überführt. Erfindungsgemäß kann es dabei vorgesehen sein, dass die erste Pericardschicht vorher in einem flüssigen Dispersionsmittel, in der Regel einem wässrigen Medium, vorgequollen wird.

In einer bevorzugten Ausführungsform wird zur Beschichtung der ersten Pericardschicht eine wässrige Dispersion von Pericardstücken auf die erste Pericardschicht aufgegossen und anschließend eine Lyophilisation durchgeführt. Alternativ oder in Kombination dazu kann die erste Pericardschicht mit einer wässrigen Dispersion von Pericardstücken besprüht werden und danach eine Lyophilisation durchgeführt werden.

Das Implantat gemäß der vorliegenden Erfindung kann allgemein zur Versorgung von Flüssigkeits- und/oder Luftleckagen im menschlichen und/oder tierischen Körper eingesetzt werden. Das Implantat kann insbesondere als Hämostyptikum verwendet werden. So eignet sich das Implantat vor allem zur Blutstillung bei inneren Wunden, Organrupturen und/oder Organresektionen. Ein weiterer Verwendungszweck betrifft die Versiegelung von Anastomosen. Eine bevorzugte Verwendung des Implantats betrifft die Versorgung von Verletzungen an der Dura Mater. So eignet sich das Implantat insbesondere als Dura-Mater-Ersatzmaterial. Besonders bevorzugt ist die Verwendung des Implantats als selbstklebendes Implantat.

Des Weiteren betrifft die vorliegende Erfindung auch die Verwendung zumindest einer ersten und zumindest einer zweiten Pericardschicht zur Herstellung des erfindungsgemäßen Implantats. Bezüglich weiterer Eigenschaften und Merkmale, insbesondere im Hinblick auf die Pericardschichten und das Implantat, wird auf die bisherige Beschreibung verwiesen.

Weitere Einzelheiten und Merkmale der Erfindung ergeben sich aus der nachfolgenden Beschreibung bevorzugter Ausführungsformen in Form einer Figur, von Beispielen und von Unteransprüchen. In diesen Ausführungsformen können einzelne Merkmale der Erfindung allein oder in Kombination mit anderen Merkmalen verwirklicht sein. Die Figur wird hiermit durch ausdrückliche Bezugnahme zum Inhalt dieser Beschreibung gemacht.

In der Zeichnung zeigt:

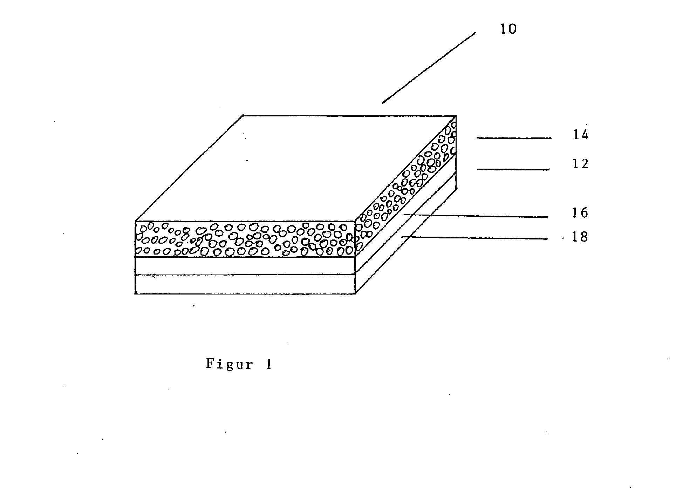

- Figur 1:: eine bevorzugte Ausführungsform des erfindungsgemäßen Implantats.

### Figurenbeschreibung

Das in Figur 1 schematisch wiedergegebene Implantat 10 weist einen zweischichtigen Aufbau aus einer ersten Pericardschicht 12 und einer zweiten Pericardschicht 14 auf. Die Pericardschichten 12 und 14 berühren sich vollflächig entlang einer gemeinsamen Grenzfläche. Die Pericardschicht 12 ist aus einem aufgearbeiteten Rinderperikardgewebe hergestellt und liegt als lyophilisierte Folie vor. Die Pericardschicht 12 weist außerdem eine Unterschicht 16 aus Pericardium fibrosum und eine Unterschicht 18 aus Pericardium serosum auf. Die zweite Pericardschicht 14 ist auf das schichtförmige Pericardium fibrosum 16 auflyophilisiert. Die Pericardschicht 12, insbesondere deren Unterschicht 16, ist von fester Beschaffenheit. Die Pericardschicht 12 dient zum einen als stabilisierende Unterlage für die zweite Pericardschicht 14. Zum anderen erlaubt die feste Struktur der ersten Pericardschicht 12 eine sichere Fixierung bzw. Verankerung des Implantats 10 im Bereich des Applikationsortes. Die zweite Pericardschicht 14 liegt als Lyophilisationsschwamm vor und ist von poröser Struktur mit einer Porengröße zwischen 20 und 150 µm. Die Pericardschicht 14 besitzt aufgrund ihrer schwammartigen und insbesondere porösen Struktur saugfähige Eigenschaften. Die zweite Pericardschicht 14 dient daher insbesondere zum Aufsaugen von Körperflüssigkeiten, wodurch die Pericardschicht 14 normalerweise hydrogelartige Eigenschaften erhält, die mit besonderem Vorteil eine Verklebung oder Versiegelung der Aplikationsstelle, beispielsweise eines Wundareals, bewirken.

### Beispiel 1: Aufarbeitung von rohem Pericard-Gewebe

### 1.1. Rohstoff-Gewinnung

Die als Ausgangsmaterial dienenden Herzbeutel (Pericardium) von Rindern werden im Schlachthof nach der üblichen Fleischbeschau durch einen amtlichen Tierarzt zunächst von anhaftenden Organteilen abgetrennt und grob von Fett und Bindegewebe befreit. Man erhält auf diese Weise flächige Stücke von der ungefähren Größe 30 x 15 cm und einem Stückgewicht von etwa 1 kg. Diese so vorbereiteten Rinderherzbeutel werden dann in einer mit Eis beschickten Kühltasche vom Schlachthof zur Produktionsstätte transportiert und dort je nach Menge des anfallenden Rohmaterials vor der Weiterverarbeitung bei unter -120 °C zwischengelagert.

### 1.2. Nasschemische Aufbereitung

Die rohen Pericard-Stücke werden zunächst einzeln mit gereinigtem Wasser gespült, üblicherweise fließend gewässert, um anhaftendes Blut und wasserlösliche Eiweißanteile zu entfernen. Nach dem Wässern werden alle makroskopisch sichtbaren Reste von Fettgewebe und Basalmembranen entfernt. Anschließend erfolgt eine Behandlung mit 2%iger wässriger Natronlauge bei Raumtemperatur. Die Pericardstücke (5000 g) verbleiben insgesamt 16 Stunden im Laugenbad (37,5 l). Nach Entnahme erfolgt dann ein ca. 10 Minuten dauernder Spülprozess in entmineralisiertem Wasser, der so oft wiederholt wird, bis der pH des Spülwassers auf einen Wert unter 8 gesunken ist. Dies ist nach ca. 1 Stunde erreicht. Sollten noch Basalmembranen und Fettreste festzustellen sein, so werden sie auf dieser Prozessstufe endgültig entfernt. Die stark gequollenen Pericard-Stücke werden nun in 37,5 l einer 10%igen wässrigen Kochsalzlösung transferiert, um den für die weiteren Prozessschritte erforderlichen Quellzustand (partielle Entquellung) einzustellen. Die Natriumchloridbehandlung wird bei Raumtemperatur durchgeführt. Es schließt sich ein Spülprozess mit entmineralisiertem Wasser an. Um störende Schwermetallionen und etwaige Kalkeinlagerungen aus dem Pericardgewebe zu entfernen, erfolgt dann eine Behandlung mit 37,5 l schwach alkalisch eingestellter EDTA-Lösung (Zusammensetzung pro 100 ml: 0,3 g). Anschließend wird wie in den vorausgegangenen Prozessschritten mit entmineralisiertem Wasser gespült, um überschüssigen Komplexbildner zu entfernen und gleichzeitig den pH auf einen Wert von 8,5 zu bringen. Die nun folgende Behandlung mit 1 x 37,5 l Acetatpuffer (pH = 4,8; Zusammensetzung pro 100 ml: 0,01 mol pro 100 ml Natriumacetat, 3 H₂O, 59 Vol.-Teile; 0,01 mol pro 100 ml Essigsäure, 41 Vol.-Teile) dient dem Zweck, alle etwa noch im Pericardgewebe verbliebenen Laugenreste abzupuffern und ein schwach saures Milieu für den späteren Bleichprozess vorzubereiten. Überschüssige Puffersubstanzen werden wie vorstehend beschrieben durch Spülen mit entmineralisiertem Wasser entfernt.

### 1.3. Oxidative Bleichungen

Im Anschluss an die nasschemische Aufbereitung werden die Pericardstücke einer einstündigen oxidativen Bleichung in 37,5 I 1,5%iger Wasserstoffperoxidlösung unterworfen. Wie alle vorausgegangenen Behandlungsschritte wird auch der Bleichprozess bei Raumtemperatur durchgeführt. Auf diese Weise wird einerseits die Effizienz der Reinigungsoperation sichergestellt, andererseits aber eine Beeinträchtigung des kollagenen Gewebes vermieden.

### 1.4. Auswaschen

Zur Entfernung überschüssiger Reagenzien wird anschließend mit entmineralisiertem Wasser gespült.

### 1.5. Entfettung

Die gespülten Rinder-Pericard-Stücke werden in eine solche Menge Aceton gelegt, dass das Rinder-Pericard-Gewebe vollständig mit Aceton überdeckt ist. Innerhalb von 8 Stunden wird das Lösungsmittel dreimal erneuert. Die so entwässerten Rinder-Pericard-Gewebe werden daraufhin in eine Soxhlet-Apparatur überführt und ca. 7 Stunden mit Aceton extrahiert. Nach der Extraktion werden die Pericard-Stücke an der Luft getrocknet und anschließend in einem Transport-Gefäß mit endmineralisiertem Wasser rehydratisiert.

### 1.6. Gefriertrocknung

Die Trocknung erfolgt in einer automatisch gesteuerten Gefriertrocknungsanlage. Die Gefriertrocknung verläuft im Einzelnen wie folgt: Temperaturabsenken auf + 1 °C, Temperaturabsenken auf -40 °C, Einschalten des Vakuums, Aufheizen der Stellplatten auf + 40 °C und Trocknen bei vollem Vakuum.

### 1.7. Sterilisierung

Die Sterilisierung erfolgt durch Strahlensterilisation mit ca. 2,5 Mrad.

### 2. Herstellung des Implantats

Zur Herstellung des Implantats werden Rinder-Pericard-Stücke in endmineralisiertem Wasser vorgequollen und anschließend mit einem Dispergiergerät (Ultra-Turrax) in wässrigem Medium mechanisch zerkleinert. Durch die Messer des Dispergiergerätes werden die Kollagenfibrillen aus dem Pericardgewebe "herausgeschält".

Ein entsprechend Beispiel 1 aufgearbeitetes Pericardgewebe wird für 5 bis 10 Minuten in entmineralisiertem Wasser eingelegt. Anschließend wird das vorgequollene Pericardgewebe derart auf eine Gefriertrocknungsplatte aus Stahl aufgelegt, dass das Pericardium fibrosum nach oben zeigt. Danach wird die Pericard-Dispersion auf das aufgelegte Pericardgewebe gegossen. Anschließend wird lyophilisiert. Durch die Lyophilisation erhält man ein zweischichtiges Pericard-Implantat mit einer mechanisch festen und widerstandsfähigen ersten Pericardschicht und einer schwammartigen zweiten Pericardschicht.

## Patentansprüche

1. Implantat (10), insbesondere zur Wiederherstellung und/oder Regenerierung von menschlichem und/oder tierischem Gewebe, mit einem schichtförmigen Aufbau, umfassend zumindest eine erste Pericardschicht (12) und eine zweite Pericardschicht (14), **dadurch gekennzeichnet, dass** die erste Pericardschicht (12) folienartig und die zweiten Pericardschicht (14) schwammartig ausgebildet ist.

2. Implantat (10) nach Anspruch 1, **dadurch gekennzeichnet, dass** sich die erste Pericardschicht (12) und die zweite Pericardschicht (14) entlang einer gemeinsamen Grenzfläche berühren.

3. Implantat (10) nach Anspruch 1 oder 2, **dadurch gekennzeichnet, dass** die erste Pericardschicht (12) eine Unterschicht (16) aus Pericardium fibrosum und eine Unterschicht (18) aus Pericardium serosum aufweist, wobei bevorzugt die Unterschicht (16) aus Pericardium fibrosum auf einer Seite mit der zweiten Pericardschicht (14) belegt ist.

4. Implantat (10) nach einem der vorhergehenden Ansprüche, **dadurch gekennzeichnet, dass** die zweite Pericardschicht (14) aus vorzugsweise homogenisierten Pericardstücken gebildet ist.

5. Implantat (10) nach einem der vorhergehenden Ansprüche, **dadurch gekennzeichnet, dass** die erste Pericardschicht (12) und/oder zweite Pericardschicht (14) lyophilisiert vorliegen.

6. Implantat (10) nach einem der vorhergehenden Ansprüche, **dadurch gekennzeichnet, dass** die erste Pericardschicht (12) eine lyophilisierte Pericardfolie und die zweite Pericardschicht (14) eine schwammartige, lyophilisierte Pericardschicht ist.

7. Implantat (10) nach einem der vorhergehenden Ansprüche, **dadurch gekennzeichnet, dass** das Pericard der ersten Pericardschicht (12) und/oder zweiten Pericardschicht (14) xenogenen, insbesondere bovinen, porcinen und/oder equinen, Ursprungs ist.

8. Implantat (10) nach einem der vorhergehenden Ansprüche, **dadurch gekennzeichnet, dass** die erste Pericardschicht (12) einen Anteil zwischen 20 und 80 Gew.-%, insbesondere 40 und 60 Gew.-%, bezogen auf das Gesamtgewicht des Implantats (10), aufweist.

9. Verfahren zur Herstellung eines Implantats (10) nach einem der vorhergehenden Ansprüche, wobei zumindest eine erste Pericardschicht (12) mit einer zweiten Pericardschicht (14) beschichtet wird.

10. Verfahren nach Anspruch 9, **dadurch gekennzeichnet, dass** für die Beschichtung der ersten Pericardschicht (12)
a) die erste Pericardschicht (12) mit einer flüssigen Dispersion, umfassend Pericardstücke und ein Dispersionsmittel, in Kontakt gebracht wird und
b) anschließend das Dispersionsmittel unter Ausbildung der zweiten Pericardschicht entfernt wird.

11. Verwendung zumindest einer ersten Pericardschicht (12) und einer zweiten Pericardschicht (14) zur Herstellung eines Implantats (10) nach einem der Ansprüche 1 bis 8.

## Claims

1. Implant (10), in particular for restoration and/or regeneration of human and/or animal tissue, having a layered structure, comprising at least a first pericardium layer (12) and a second pericardium layer (14), **characterized in that** the first pericardium layer (12) has a film-like structure and the second pericardium layer (14) has a sponge-like structure.

2. Implant (10) according to claim 1, **characterized in that** the first pericardium layer (12) and the second pericardium layer (14) contact along a common interface.

3. Implant (10) according to claim 1 or 2, **characterized in that** the first pericardium layer (12) has a sublayer (16) composed of pericardium fibrosum and a sublayer (18) composed of pericardium serosum, wherein preferably the sublayer (16) composed of pericardium fibrosum is covered by the second pericardium layer (14) on one side.

4. Implant (10) according to any one of the preceding claims, **characterized in that** the second pericardium layer (14) is composed of preferably homogenized pieces of pericardium.

5. Implant (10) according to any one of the preceding claims, **characterized in that** the first pericardium layer (12) and/or the second pericardium layer (14) are/is present in a lyophilized condition.

6. Implant (10) according to any one of the preceding claims, **characterized in that** the first pericardium layer (12) is a lyophilized pericardium film and the second pericardium layer (14) is a sponge-like, lyophilized pericardium layer.

7. Implant (10) according to any one of the preceding claims, **characterized in that** the pericardium of the first pericardium layer (12) and/or the second pericardium layer (14) are/is of xenogenic origin, in particular of bovine, porcine and/or equine origin.

8. Implant (10) according to any one of the preceding claims, **characterized in that** the first pericardium layer (12) has a proportion of between 20 and 80 % by weight, in particular 40 and 60 % by weight, in relation to the total weight of the implant (10).

9. Method for producing an implant (10) according to any one of the preceding claims, wherein at least a first pericardium layer (12) is coated with a second pericardium layer (14).

10. Method according to claim 9, **characterized in that** for coating the first pericardium layer (12)
a) the first pericardium layer (12) is contacted with a liquid dispersion, comprising pieces of pericardium and a dispersant, and
b) subsequently the dispersant is removed while the second pericardium layer is developing.

11. Use of at least a first pericardium layer (12) and a second pericardium layer (14) for producing an implant (10) according to any one of the claims 1 to 8.

## Revendications

1. Implant (10), notamment pour la restauration et/ou la régénération de tissu humain et/ou animal, avec une structure de type stratifiée, comprenant au moins une première couche péricardique (12) et une deuxième couche péricardique (14), **caractérisé en ce que** la première couche péricardique (12) est réalisée en forme de feuille et la deuxième couche péricardique (14) est réalisée en forme de mousse.

2. Implant (10) selon la revendication 1, **caractérisé en ce que** la première couche péricardique (12) et la deuxième couche péricardique (14) se touchent le long d'une surface limite commune.

3. Implant (10) selon la revendication 1 ou 2, **caractérisé en ce que** la première couche péricardique (12) présente une sous-couche (16) en pericardium fibrosum et une sous-couche (18) en pericardium serosum, dans lequel la sous-couche (16) en pericardium fibrosum est de préférence recouverte sur un côté par la deuxième couche péricardique (14).

4. Implant (10) selon l'une quelconque des revendications précédentes, **caractérisé en ce que** la deuxième couche péricardique (14) est formée de morceaux de péricarde de préférence homogénéisés.

5. Implant (10) selon l'une quelconque des revendications précédentes, **caractérisé en ce que** la première couche péricardique (12) et/ou la deuxième couche péricardique (14) est/sont présente(s) sous forme lyophilisée.

6. Implant (10) selon l'une quelconque des revendications précédentes, **caractérisé en ce que** la première couche péricardique (12) est une feuille de péricarde lyophilisée et la deuxième couche péricardique (14) est une couche péricardique sous forme de mousse lyophilisée.

7. Implant (10) selon l'une quelconque des revendications précédentes, **caractérisé en ce que** le péricarde de la première couche péricardique (12) et/ou de la deuxième couche péricardique (14) est d'origine xénogène, en particulier d'origine bovine, porcine et/ou équine.

8. Implant (10) selon l'une quelconque des revendications précédentes, **caractérisé en ce que** la première couche péricardique (12) représente une proportion comprise entre 20 et 80 % en poids, en particulier entre 40 et 60 % en poids, rapportée au poids total de l'implant (10).

9. Procédé de fabrication d'un implant (10) selon l'une quelconque des revendications précédentes, dans lequel au moins une première couche péricardique (12) est revêtue avec une deuxième couche péricardique (14).

10. Procédé selon la revendication 9, **caractérisé en ce que**, pour le revêtement de la première couche péricardique (12)
a) la première couche péricardique (12) est mise en contact avec une dispersion liquide, comprenant des morceaux de péricarde et un agent de dispersion, et
b) ensuite, l'agent de dispersion est éliminé avec formation de la deuxième couche péricardique.

11. Utilisation d'au moins une première couche péricardique (12) et d'une deuxième couche péricardique (14) pour la fabrication d'un implant (10) selon l'une quelconque des revendications 1 à 8.
